# EUROPEAN PATENT SPECIFICATION

(11) **EP 4 658 341 B1**
(45) Date of publication and mention of the grant of the patent: **30.09.2026**
(21) Application number: 23836844.3
(22) Date of filing: 21.12.2023
(51) Int. Cl.: A61M 5/32, A61M 5/20

(54) **SAFETY NEEDLE COVER LOCK FOR AN AUTOINJECTOR**
SICHERHEITSNADELABDECKUNGSVERRIEGELUNG FÜR EINEN AUTOINJEKTOR
VERROU DE PROTECTION D'AIGUILLE DE SÉCURITÉ POUR AUTO-INJECTEUR

(30) Priority: 01.02.2023 EP 23154530
(43) Date of publication of application: 10.12.2025
(73) Proprietor: Ypsomed AG, 3401 Burgdorf (CH)
(72) Inventor: TSCHIRREN, Markus, 3400 Burgdorf (CH); GRUENIG, Nicolas, 1784 Courtepin (CH); KALBERMATTER, Gabriel, 3400 Burgdorf (CH)
(86) International application number: PCT/EP2023/087170
(87) International publication number: WO 2024/160450

(56) References cited:
- WO-A1-2022/078986
- US-A1- 2015 174 325
- US-A1- 2020 061 302

## Description

### FIELD OF THE INVENTION

The present invention relates to drug delivery devices or medicament delivery devices for injecting, delivering, administering or dispensing substances and/or liquids such as insulin, hormone preparations or vaccines. It departs from a needle cover lock for an autoinjector which is adapted to automatically dispense a liquid product through a needle of a prefilled syringe. The needle cover lock comprises a housing to hold the syringe, and a needle cover sleeve movable relative to the housing between an extended position and a retracted position. The needle cover lock further includes a removably attached device cap at a distal end of the autoinjector covering a distal end of the cover sleeve in an unused state of the autoinjector.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection devices. An injection device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the injection device. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with prefilled syringes. The majority of autoinjectors are configured as single use devices which incorporate both the prefilled syringe and the drive mechanism in the same housing. Such devices are usually disposable for hygiene reasons.

Autoinjectors usually comprise a body for housing the syringe as well as an automatic drive mechanism in order to move the plunger of the syringe upon actuation of the autoinjector. The drive mechanism of the autoinjector typically comprises a source of drive, such as a motor or strong spring for moving a transfer element, for example a rod, which acts on the plunger of the syringe. The prefilled syringe has a needle or cannula that is permanently attached, glued, or otherwise staked to a first end of a container or reservoir that is sealed at the opposing end by the plunger.

For safety and hygiene reasons it is desirable that the needle does not protrude from a housing of the autoinjector with the exception of the time when the needle is used for injection of a medicament. Thus, either the autoinjector moves the needle out of the housing for the injection and back into the housing after injection or the autoinjector includes a needle cover or needle guard which may be moved from an extended position into a retracted position to unsheathe the needle for injection and which may be moved back in a needle covering position after the injection. **In** the extended or in an initial position the needle cover may cover or shield the needle. Alternatively, the extended or initial position is used only as a starting point before the needle cover is moved towards the retracted position to trigger a start of the dispensing mechanism of the autoinjector. Hybrid configurations with needle and needle guard being moved once and in the same direction are also conceivable.

Besides the needle cover such autoinjector usually feature a device cap attached to a distal end in a shipping state or unused condition. The cap may be used to remove a sterile barrier in form of so-called rigid needle shield (RNS) initially shielding the needle and keeping the needle sterile prior use.

It has to be ensured that the needle is covered and the sterile barrier is reliably held onto the needle during shipping and prior use of the autoinjector. For that purpose, the autoinjector usually includes a safety release lock or needle cover lock. If the needle cover serves to trigger the injection when being pushed to the retracted position, unintended triggering prior to removal of the device cap is prevented by a needle cover lock.

US 2012/0203186 A1 discloses an autoinjector with a sleeve-shaped housing, a needle protection sleeve movable relative to the housing. The protection sleeve further includes elastic arms engaging a portion of the housing. A needle shield is detachably fastened to an injection needle to keep the needle sterile. The needle shield is further connected to a removable device cap. The device cap includes a sleeve-shaped holding portion situated on an inner circumference of the protection sleeve to prevent the elastic arms from moving radially inwards, and thus ensures that the protection sleeve is prevented from moving relative to the housing in the autoinjector as shipped, i.e. with the device cap attached. The elastic arms thus serve to provide a secure transport. If the device cap is removed the elastic arms are enabled to move radially inwards and thus the protection sleeve can be moved in a retracted position.

WO08113864 A1 discloses an autoinjector comprising a cover sleeve. The cover sleeve includes integrally formed flexible locking arms that can be deflected radially inwards to the longitudinal axis of the autoinjector. The locking arms are hold in a blocking position by a device cap. When the cap is removed the flexible locking arms can deflect and thereby the cover sleeve is unlocked and can be moved into a retracted position.

WO 2016/193352 A1 discloses an autoinjector with a needle shroud movable relative to a device housing. If a cap is removed from the autoinjector a protective needle sheath is removed from a needle of a syringe. The needle shroud is then free to move in a retracting direction and the autoinjector is ready to use. If the autoinjector is removed from the injection site after the injection, the needle shroud moves back into a covering direction. A shroud beam deflects radially inwards due to a tapered inner diameter of a front part. After passing a stop the shroud beam relaxes radially outwards abutting the stop which is possible as the cap is not present at that time. Afterwards, the shroud beam cannot return in the retracting direction as it would hit the stop. The needle shroud is thus locked in a covering position.

These prior art approaches focus on radially deflectable elements locking or unlocking the needle cover sleeve relative to a housing. Such locking mechanisms may suffer from a faulty unlocking of the cover sleeve, in particular if the deflectable locking elements stick on the housing or if the cap and thus the cover sleeve is not correctly released.

Further possibilities to lock a cover sleeve as long as a protective cap is attached to the device are known from WO 2022/078986 A1, US 2015/174325 A1 and US 2020/061302 A1.

### DESCRIPTION OF THE INVENTION

It is an objective of the invention to provide a reliable locking and unlocking of a needle cover of an autoinjector.

This objective is achieved by the needle cover lock according to the independent claim. Preferred embodiments are evident from the dependent claims.

According to the invention a needle cover lock for an autoinjector is provided. The autoinjector is adapted to automatically dispense a liquid product through a needle of a prefilled syringe. The needle cover lock comprises
- a housing adapted to hold the syringe non-movable and defining a longitudinal direction;
- a needle cover sleeve movable relative to the housing between an extended position and a retracted position;
- a device cap attached to a distal end of the autoinjector and covering a distal end of the autoinjector and thus of the cover sleeve in an unused state or shipping state of the autoinjector. The device cap is removable by the user prior to use of the autoinjector.

The housing or the cover sleeve includes a flexible locking member and the other of the housing and cover sleeve includes a stop surface. The device cap includes a blocking surface preventing a deflection of the locking member together with the stop surface. **In** this state the stop surface is preferably on a first side of the locking member and the blocking surface is preferably on a second side of the locking member opposite the first side at the same time. Therefore, the cover sleeve is prevented to exit the extended position or covering position. If the device cap with its blocking surface is removed from the autoinjector the locking member is allowed to deflect (preferably on the second side of the locking member) thereby allowing the needle cover to exit the extended position or covering position and to move towards and into the retracted position. The locking member is deflectable (preferably on the second side and also on the first side) in a plane tangent to a circumferential outer surface of the cover sleeve if the device cap is removed from the autoinjector.

The arrangement with the locking member, the stop surface and the blocking surface reliably holds the cover sleeve in the extended position. As the flexible locking member is not deflectable radially but exclusively deflectable in a plane tangent to the outer surface of the cover sleeve a proper working of the deflection is enabled as in the tangential plane no other element impedes the travel path of the locking member. This is different from a locking member deflectable radially inwards as known in the art which may get in contact with inner elements of the autoinjector which elements may hinder a proper deflection.

Furthermore, the arrangement according to the invention with the tangential deflectable locking member facilitates a guiding of the locking member as in the tangential plane no interaction with other elements of the autoinjector (e.g. needle, syringe, needle shield) is to be expected.

The needle cover lock according to the invention provides thus for reliable locking and in particular a reliable unlocking of the cover sleeve. Hence, the cover sleeve can be moved from a extended or covering position into the retracted position and thereby prepare the autoinjector for an upcoming injection.

In a preferred embodiment the extended position is a needle covering position in which the needle is covered by the cover sleeve. Correspondingly, in the retracted position the needle cover is no longer covering the needle and thus the needle is exposed. However, in an alternative embodiment the cover sleeve can be used exclusively to activate the dispensing mechanism by moving the cover sleeve from the extended position to the retracted position. This means in this embodiment the needle cover does not mandatorily has to cover the needle in the extended position. A shielding of the needle may be provided by the device cap or a further component of the autoinjector.

The device cap is mounted on a distal end of the autoinjector in a shipping state or unused state of the autoinjector. In order to prepare the autoinjector for an injection the user has to remove the cap from the distal end. Thus, when the cap is removed and thus the blocking surface is removed the locking member is no longer hindered to deflect, preferably it can deflect in a plane tangent to the outer surface of the cover sleeve on the side of the second face of the locking member. The deflection allows a relative movement between the locking member and the stop surface and thus the cover sleeve is no longer prevented to exit the covering position and to be moved towards and into the retracted position.

The stop surface may be arranged on the housing and the deflectable member may be arranged on or integrally formed in the cover sleeve. In an alternative embodiment the stop surface may be provided by the cover sleeve and the locking member may be arranged on the housing.

The device cap includes the blocking surface. That means the blocking surface is arranged on a cap element, fixedly connected to the cap or integral to the cap as a monolithic part of the cap.

The stop surface and the blocking surface both together are thus adapted to prevent any movement of the locking member, in particular any deflection, and hold the locking member in an initial position if the device cap is attached to the autoinjector. That means the locking member is only deflectable from its initial position if the device cap and thus the blocking surface is removed from the autoinjector. As the drive and thus the dispensing of the autoinjector can only be activated if the cover sleeve is in its retracted position the prevention of movement of the cover sleeve out of its covering position prevents an unintentional activation of the drive of the autoinjector.

The locking member is deflectable in a plane tangent to a circumferential surface of the cover sleeve (e. g. tangent to a cylindrical shape of the cover sleeve). That means the locking member does not deflect exclusively radially inwards towards a center of the autoinjector or in a plane including the device axis but instead it can deflect in a plane parallel to the longitudinal axis and tangent to the cylindrical shape of the cover sleeve. The locking member may deflect along a circumferential direction such that only a movement component is in the plane tangent to the cover sleeve.

The locking member may be, for example, a flexible arm, a hook, a protrusion, a pin or the like. The locking member is preferably made of an elastic material such as plastic or metal. Furthermore, the locking member may be a com rotatable but axially fixed with respect to the cover sleeve. The locking member may be deflectable around a hinge with an axis perpendicular to the longitudinal axis of the housing.

The autoinjector is adapted to automatically dispense a liquid product through the needle of the prefilled syringe. The autoinjector is preferably adapted to dispense the whole liquid in one single stroke or shot. The prefilled syringe held by the autoinjector includes a needle which is permanently and non-removably attached to a syringe body or barrel.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

Preferably, the stop surface is arranged on a rib, which extends along the longitudinal direction and which is adapted to guide the locking member during a movement of the locking member relative to the rib. Thus, the locking member may be in contact with the rib during the movement when the cover sleeve is moved from the covering position towards the retracted position.

The housing is preferably sleeve-shaped and the stop surface is preferably arranged on an inner surface of the sleeve. In this embodiment, the locking member is provided by the cover sleeve and thus the cover sleeve may be arranged inside the sleeve-shaped housing. This provides for a compact design. Furthermore, as the locking member is covered or encompassed by the sleeve-shaped housing the locking member is protected from external influences, e.g. an unintentional actuation of the locking member can reliably be prevented.

A sloped surface is preferably arranged on a distal end of the rib, preferably a distally faced sloped surface. Therefore, the locking member may abut or engage the sloped surface on the end of the rib if the locking member is moved relative to the rib. The sloped surface may facilitate the deflection of the locking member upon movement of locking member relative to the rib. As the sloped surface helps to deflect the locking member a reliable unlocking of the cover sleeve can be provided.

In a preferred embodiment the device cap includes a blocking rib extending in the longitudinal direction. The blocking surface is arranged on a longitudinal side of the blocking rib. This provides for a compact design as the blocking rib may be integrally formed in the device cap.

The blocking rib is further preferably arranged inside the device cap. This provides for a compact design too. The device cap is usually sleeve-shaped or has a cylindrical shape with an opening on a proximal side wherein the blocking rib may be arranged inside the opening.

In a preferred embodiment the locking member has a sloped contact surface (preferably proximally faced) to facilitate deflection of the locking member upon movement of the locking member relative to the stop surface if the cover sleeve is moved from the covering position towards the retracted position. The sloped contact surface facilitates and guides the deflection of the locking member and thus enables a reliable release of the cover sleeve from the covering position.

The locking member is preferably provided by the cover sleeve. In particular, the locking member is integrally formed in the cover sleeve. That means the locking member is a monolithic part of the cover sleeve and made of the same material as the cover sleeve. This provides for a cost-saving manufacturing.

The locking member is preferably a flexible arm extending in the longitudinal direction. Preferably, a first and second face of the locking member extend each on a longitudinal side of the arm. An arm-shaped locking member can be easily deflected and guided during the movement of the cover sleeve relative to the housing. Preferably, the arm is deflectable around an axis perpendicular to a longitudinal axis of the housing.

The flexible arm preferably includes a knob portion at a free end of the arm. The knob portion helps to reliably guide and to facilitate deflection of the arm upon contact with a counter element, for example rib and/or a sloped contact surface.

In a preferred embodiment the needle cover lock includes a first and second flexible arm parallel arranged to each other and the device cap includes a first blocking surface for the first arm and second blocking surface for the second arm. The first and second blocking surfaces are oppositely arranged to each other and preferably in-between the two parallel arms if the device cap is attached to the autoinjector. If the device cap includes a blocking rib the first and second blocking surfaces are preferably each arranged on a longitudinal side of the blocking rib.

In this embodiment the needle lock further includes a first stop surface for the first flexible arm and a second stop surface for the second arm. Hence, if the cap is attached the first stop surface and first blocking surface prevent the first arm from deflecting and correspondingly the second stop surface and the second blocking surface prevent the second arm from deflecting. If the cap is removed from the autoinjector the first and second arm can deflect towards each other and thus the cover sleeve is released and can be moved towards the retracted position.

The embodiment including two arms may increase the reliability regarding the locking and unlocking of the cover sleeve. Upon removal of the cap the first and second blocking surface are removed and the arms are allowed to deflect on the side where the blocking surfaces were previously present.

In a preferred embodiment in the covering position the attached device cap and the cover sleeve are both movable together relative to the housing, preferably a short distance, in particular less than 5mm.

The blocking surface or the stop surface may be implemented such that the locking member can at least a short distance (e. g. several millimeters) travel along the blocking surface. For example, the stop surface and the blocking surface may extend in the longitudinal direction or may be arranged on a longitudinal element. That means the stop surface and the blocking surface may prevent a deflection of the locking member if the cap is mounted but may allow a movement in the longitudinal direction between the cap and the housing and/or between the cover sleeve and the housing.

In other words, there may be a play in the longitudinal direction between the cap and the housing and/or between the cover sleeve and the housing if the cover sleeve is in the covering position. This play or gap facilitates the assembly and may compensate for material and manufacturing tolerances.

Furthermore, if the autoinjector experiences a force, for example, if it is dropped or hit during shipping or packaging, the cap and the cover sleeve may absorb a part of the acting forces which may prevent damages.

Preferably, the cover sleeve is biased by a cover spring, which biases the cover sleeve into the covering position. That means the cover sleeve has to be pushed against a force of the cover spring when it is moved from to the covering position towards the retracted position. The cover spring ensures that the cover sleeve does not unintentionally exit the covering position.

The invention further relates to an autoinjectors with the need cover lock as described above. In a preferred embodiment the autoinjector includes the housing, an automatic drive with a plunger rod and a drive member, for example a pretensioned spring, for automatically moving the plunger rod in a dispensing direction to dispense the liquid product from the syringe.

Furthermore, the housing is preferably adapted to hold the syringe in an axially non-movable manner. That means the syringe cannot be moved relative to the housing during an injection. This distinguishes the autoinjector according to the invention from autoinjectors that support the syringe axially movable to move the syringe relative to the housing, for example, to penetrate the needle into the injection site.

The disclosure relates further to a method to unlock a needle cover lock of an autoinjector, wherein the autoinjector comprises a prefilled syringe with a needle. The method comprising the steps of
a) Removing a device cap from a distal end of the autoinjector and thereby removing a blocking surface from a locking member;
b) Moving, in a longitudinal direction, a needle cover sleeve from a needle covering position in which the needle is covered towards a retracted position in which the needle is exposed and thereby engaging the locking member of the cover sleeve with a stop surface of a housing of the autoinjector;
c) deflecting, by the movement of the cover sleeve, the locking member in a plane tangent to an outer surface of the cover sleeve and thereby allowing further movement of the cover sleeve towards the retracted position.

The disclosure relates a further embodiment of a needle cover lock for an autoinjector. The autoinjector is adapted to automatically dispense a liquid product through a needle of a prefilled syringe. The further embodiment of the needle cover lock comprises
- a housing adapted to hold the syringe non-movable and defining a longitudinal direction;
- a needle cover sleeve movable relative to the housing between an extended position and a retracted position;
- a device cap attached to a distal end of the autoinjector and covering a distal end of the autoinjector and thus of the cover sleeve in an unused state or shipping state of the autoinjector. The device cap is removable by the user before using the autoinjector.

The housing or the cover sleeve includes a flexible locking member and the other of the housing and cover sleeve includes a stop surface, and wherein the device cap includes a cam surface and wherein when the device cap is attached, the cam surface together with the stop surface are adapted to restrict a movement or deflection of the locking member such that the cover sleeve is prevented to exit the extended position. When the device cap with its cam surface is removed from the autoinjector the locking member is allowed to move or to deflect such that the needle cover sleeve can exit the extended position and move towards the retracted position. The locking member is movable or deflectable in a plane tangent to a circumferential outer surface of the cover sleeve.

The device cap includes the cam surface which is adapted to deflect or move at least a part of the locking member when the locking member is moved along the longitudinal direction. The cam surface may be arranged on a cap element, fixedly connected to the cap or integral to the cap as a monolithic part of the cap.

The cam surface and the blocking surface both together are thus adapted to restrict a movement (but allowing a short travel up to several millimeters or a play) of the locking member, in particular are adapted to prevent a deflection, and hold the locking member in an initial position if the device cap is attached to the autoinjector. That means the locking member is only movable (except a short distance of several millimeters) along the longitudinal direction from its initial position if the device cap and thus the cam surface are removed from the autoinjector. As the drive and thus the dispensing of the autoinjector can only be activated if the cover sleeve is in its retracted position the prevention of movement of the cover sleeve out of its covering position prevents an unintentional activation of the drive of the autoinjector.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of an autoinjector with a needle cover lock in an initial state according to the invention;
- Fig.2: depicts a sectional view of the autoinjector in the initial state;
- Fig.3: depicts a sectional view of the autoinjector wherein the plane for the sectional view is 90° rotated compared to the view in figure 2;
- Fig.4: depicts a sectional view of a distal portion of the autoinjector wherein the cut for the sectional view is at a distance to the longitudinal axis;
- Fig. 5: depicts a perspective view of a device cap of the autoinjector;
- Fig. 6: depicts a perspective view of a needle cover sleeve of the autoinjector;
- Fig. 7: depicts a perspective view of a housing of the autoinjector;
- Fig. 8, 9: depicts a perspective view of a telescopic outer sleeve and of a proximal housing of the autoinjector;
- Fig. 10 - 12: depict the autoinjector in a state where the device cap and cover sleeve were moved relative to a housing of the autoinjector and
- Fig. 13 - 15: depict the autoinjector in a state where the cover sleeve is in a retracted position.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

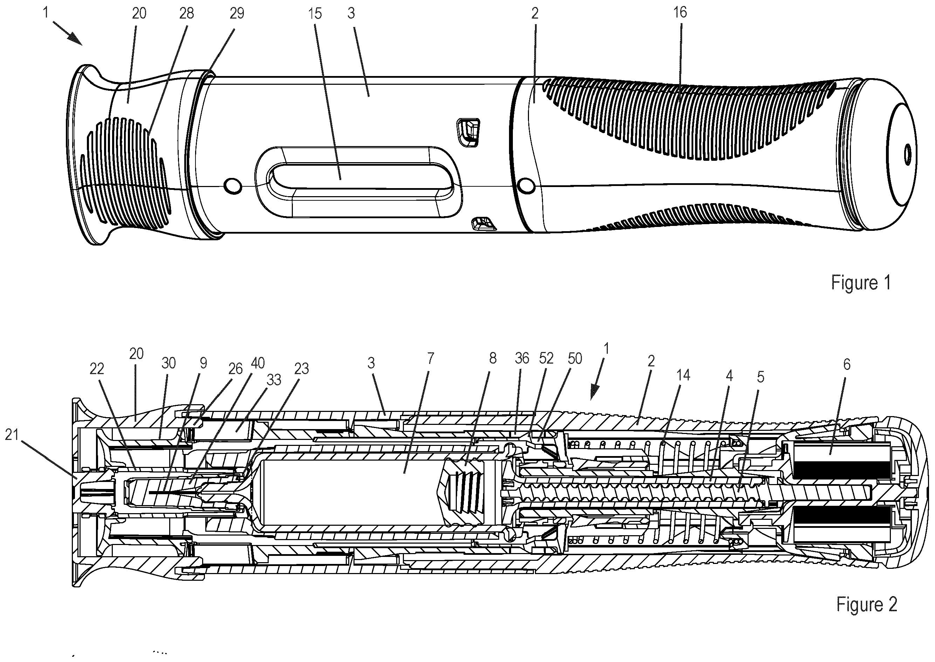

In the following the term "distal" refers to the side where an injection needle is located (e.g. left hand side in figure 1 and 2). The term "proximal" refers to the opposite side which is furthest away from the needle (right hand side in figure 1 and 2).

Figure 1 depicts a perspective view of an autoinjector 1 with a needle cover lock according to the invention. The autoinjector 1 shown in figure 1 and 2 is in an initial state or a shipping state. As it can be seen in these figures the autoinjector is cylindrically shaped and comprises a proximal housing 2 and a distal housing 3. The proximal housing 2 features a gripping structures 16 for the user to facilitate gripping the autoinjector 1. The distal housing 3 includes an opening 15 which allows the user to inspect the fill level of a syringe held inside the housing. At a distal end a cap 20 is removably attached to the distal housing 3. The cap 20 provides two cap gripping structures 28 oppositely arranged in a circumferential direction. A proximal end rim 29 of the cap varies along the circumferential direction. That means the longitudinal extension of the cap varies. Namely, in the rotational position where the opening 15 is located in the housing 3 (and where the gripping structures 28 are not present) the cap is shorter in the longitudinal direction than in a rotational position aligned with the gripping structure 28.

The following description focuses on the needle cover locking mechanism of the autoinjector 1 according to the invention. Other components and the function of the dispensing mechanism of the autoinjector 1 are described in the patent application no EP 22173096.3.

Figure 2 depicts a sectional view of the autoinjector 1 where the cut for the sectional view runs along a longitudinal axis of the autoinjector 1. As shown in figure 2 the autoinjector 1 includes a prefilled syringe 7 with a liquid drug and a needle 9, a needle cover sleeve 30, a threaded rod 5 engaging a plunger rod 4 for moving a piston 8 inside the syringe 7 to dispense the liquid drug and a drive mechanism with a prestressed drive spring 6 to drive the plunger rod 4 in the dispensing direction.

The autoinjector 1 includes means to protect the needle 9 and to avoid injuries by the needle prior use and after use of the autoinjector. As it can be seen in figure 2 the autoinjector 1 comprises the needle cover sleeve 30 which is movable along the longitudinal axis relative to the proximal housing 2 and relative to the distal housing 3. The cover sleeve 30 is biased by a cover spring 14 towards a covering position. In the state shown in figure 2 the needle cover sleeve 30 is in the covering position in which the needle 9 is shielded by the cover sleeve 30. Furthermore, in the shown state the device cap 20 is attached to a distal end of the autoinjector 1. Besides that, a rigid needle shield (RNS) 40 is mounted onto the needle 7 to protect the needle and to keep the needle 7 sterile prior use.

It is to be noted that in the context of the present invention, the type of autoinjector is not decisive. An autoinjector with a drive including a compression spring is likewise benefitting. At the same time, the cover sleeve may be in an initial extended position from which an activating stroke relative to the housing is performed, while the syringe is subsequently shifted relative to the housing. The relevant aspect is the avoidance of unintended activation or triggering by the cover sleeve.

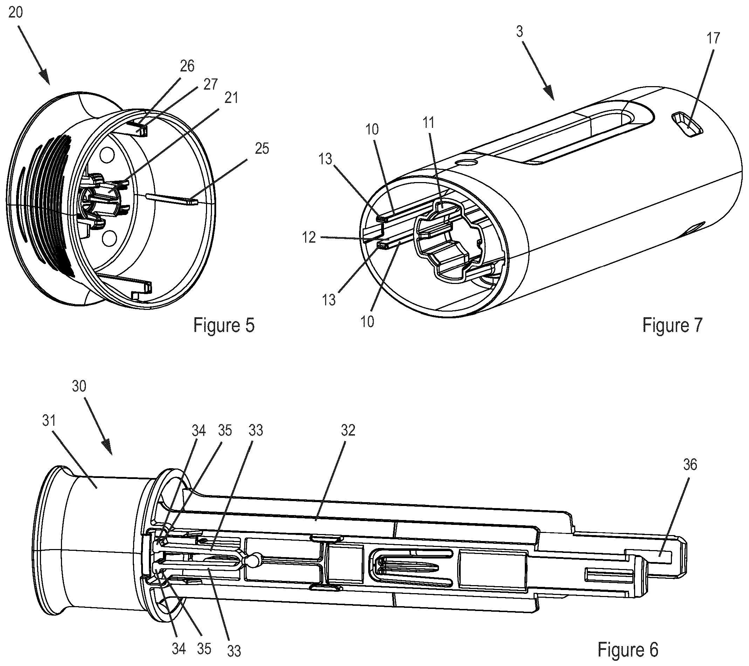

The device cap 20 comprises on a proximal end and on an inner side two oppositely arranged blocking ribs 26 forming with an outer rim a U-shaped recess for accommodating a distal end of the housing 3 of the autoinjector if the cap 20 is attached. The blocking ribs 26 are best shown in figure 5 which depicts a perspective view of the device cap 20. Each blocking rib 26 includes a first blocking 27 surface on a first longitudinal side of the blocking rib and a second blocking surface 27 on a second longitudinal side of the blocking rib 26. The first and second blocking surfaces 27 extend in the longitudinal direction and are adapted to cooperate with a flexible arm 33 of the cover sleeve 30 as described below.

Furthermore, the cap 20 comprises on its inner side two oppositely arranged protrusions 25 that provide a centering function relative to the cover sleeve 30.

In a further embodiment blocking surface may be a cam surface 27 adapted to restrict a movement or deflection of a locking arm. In this embodiment the cam surface 27 does not completely prevent a movement of the locking arm but allows a short travel. Upon contact of the locking arm with the cam surface 27 the locking arm may be deflected such that the locking arm gets in contact with a stop surface 12 and encounters the stop surface 12. The function of the stop surface 12 is described below in detail.

As shown in figure 5 the device cap 20 further comprises on its inside a support structure 21 connected to a RNS remover sleeve 22, shown in figure 2. The remover sleeve 22 encloses the RNS 40 and comprises on its proximal end hooks 23 adapted to engage a proximally oriented end of the RNS 40. The hooks 23 are thus adapted to remove the RNS 40 together with the device cap 20 if the cap is removed. As shown in figure 2 the device cap 20 surrounds the RNS 40 and a distal part of the cover sleeve 30. As shown the device cap 20 is coaxially arranged to the cover sleeve 30.

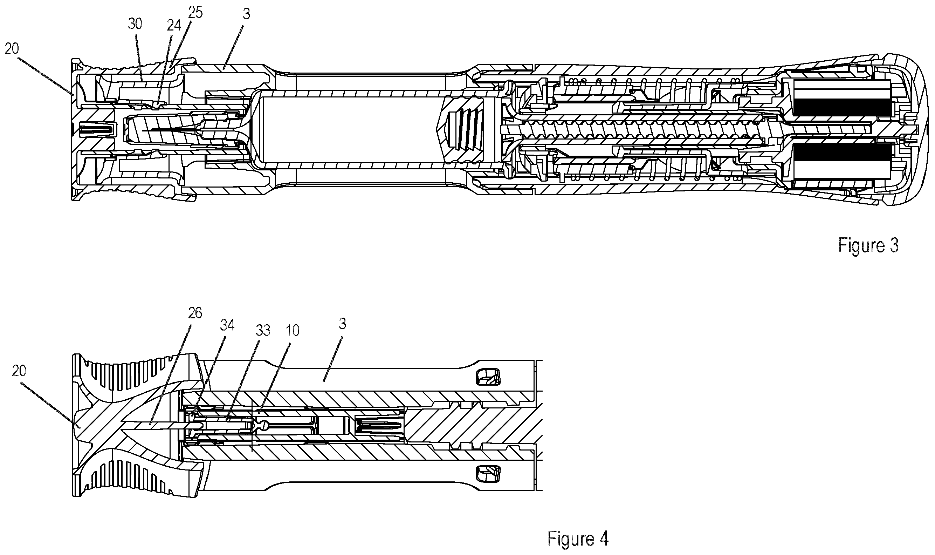

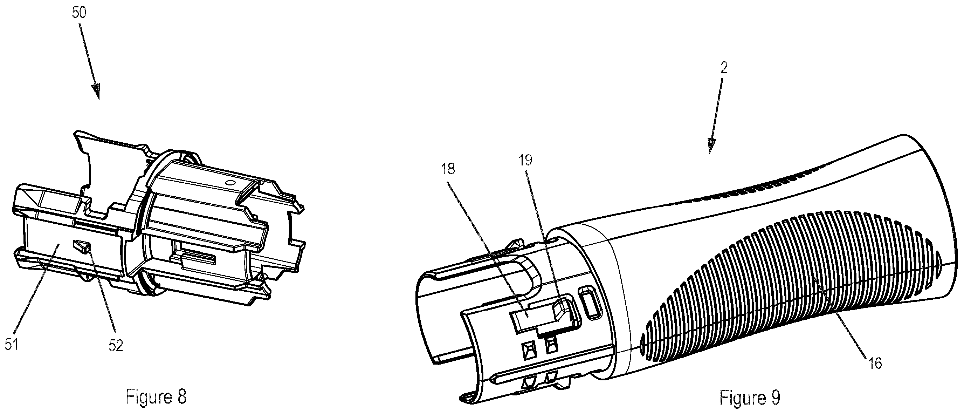

Figure 3 depicts a second sectional view of the autoinjector wherein the cut for the sectional view runs along the longitudinal axis but in a plane 90° rotated compared to the sectional view of figure 2. As shown in the sectional view of figure 3 the cover sleeve 30 comprises on its distal end arms 24 engaging in recesses in the RNS remover sleeve 22 and thus releasably connect the cover sleeve 30 to the RNS remover sleeve 22. The cap 20 is thus held on the cover sleeve 30. A perspective view of the cover sleeve 30 is depicted in figure 6. As shown in figure 6 the cover sleeve 30 comprises a sleeve-shaped distal portion 31 and two oppositely arranged and longitudinally extending shells or wings 32. On a proximal end each of the shells 32 includes an opening 36 adapted to accommodate a nose 52 of a telescopic outer sleeve as shown in figure 8.

The telescopic outer sleeve 50 is depicted in figure 8 in a perspective view. On two opposite sides the telescopic outer sleeve 50 include a contacting surface 51 with the nose 52 thereon. The cover sleeve 30 is fixedly connected to the telescopic outer sleeve 50 by a non-releasable snap-fit connection due to an engagement of the noses 52 with the openings 36 in the cover sleeve shells 32. The function of the telescopic outer sleeve is descripted in detail in the above mentioned application no EP 22173096.3.

The cover sleeve 30 further includes two pairs of flexible locking arms 33 (see figure 6) integrally formed in the shells 32. The two pairs of locking arms 33 are oppositely arranged and each locking arm 33 has a distal free end and a proximal end connected to the cover sleeve shell. Furthermore, each locking arm 33 includes an enlarged and knob-shaped end portion 34 on its free end. The knob end 34 comprises a proximally oriented sloped surface 35 to facilitate deflection of the arms 33 upon contact with a rib 10 of the housing 3. Each locking arm 33 is deflectable with its free end in a plane tangent to an outer surface of the cover sleeve 30. In other words, each flexible locking arm 33 is elastically deflectable in a direction tangent to the sleeve-shaped portion 31 of the cover sleeve 30.

Figure 4 shows a sectional view of a distal portion of the autoinjector 1. However, in contrast to figure 2 and 3 the cut for the sectional view is in a plane radially offset to the center longitudinal axis of the autoinjector. The cut for the sectional view runs through the flexible arms 33 and the blocking rib 26 of the attached device cap 20.

Figure 4 depicts the blocking rib 26 arranged in-between the two arms 33. That means the two flexible arms 33 of the cover sleeve 30 are located on both sides of the blocking rib 26. The first blocking surface 27 contacts the knob end 34 of a first locking arm 33 and the second blocking surface 27 contacts the knob end 34 of the second locking arm 33.

On an outer side of each flexible arm 33 and opposite of the blocking surface 27 a stop surface 12 is arranged on the longitudinal rib 10 of the housing 3. The rib 10 best shown in figure 7 extends radially inwards from in inside of the sleeve-shaped housing 3. Figure 7 depicts a perspective view of the housing 3. As it can be seen in figure 7 there are two parallel ribs 10 running inside the sleeve-shaped housing 3. Two pairs of ribs 10 are oppositely arranged inside the housing 3 such that for the two pair of flexible locking arms 33 two corresponding pairs of ribs 10 are provided. On its distal end each rib 10 has a sloped surface 13 facilitating the deflection of the flexible locking arm 33. The stop surface 12 extends along the rib 10 in the longitudinal direction. The sloped surface 13 at the front end of the rib 10 is intended to inhibit proximal movement of the knob 34 and the cover sleeve 30 by abutting the surface 35 of the knob. The shape and the slope of the surface 35 and of the sloped surface 13 define a break-away force level to move the cover sleeve 30 out of the covering position when the cap is removed.

In an alternative embodiment the cover sleeve may include only one pair of flexible locking arms or even only one single locking arm. Correspondingly, the housing may include only one rib adapted to engage the locking arm.

On a first side of each flexible arm 33 the rib 10 with its stop surface 12 is located and on a second side of the flexible arm 33 the blocking surface 27 of the blocking rib 26 of the device cap 20 is located. Hence, each locking arm 33 is located in-between the rib stop surface 12 and the cap blocking surface 27 (also shown in figure 4).

The distal housing 3 and the proximal housing 2 are fixedly and non-releasably connected to each other. For that purpose, the distal housing 3 comprises in a proximal end portion four circumferentially arranged openings or recess 17 as it can be seen in figure 7. The proximal housing 2 is depicted in a perspective view in figure 9. It includes in a distal portion corresponding four circumferentially arranged arms or snappers 18 each having a nose 19 on a free end. The nose 19 is adapted to snap in the recess 17 of the distal housing 3. The distal and proximal housing 2, 3 are thus fixedly connected to each other by a snap-fit connection which can be seen, for example, in figure 2.

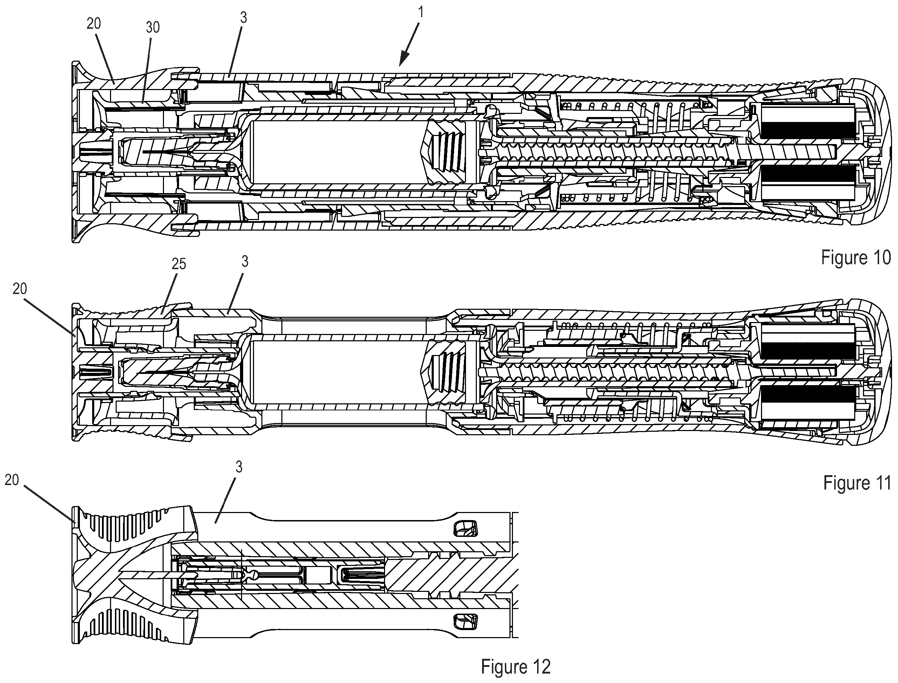

Figures 10 to 12 depict sectional views corresponding to the sectional views in figure 2 to 4. However, in the state of the autoinjector 1 shown in figures 10 to 12 the device cap 20 and the cover sleeve 30 have been moved a short distance of several millimeters relative to the housing 3. This is possible due to a gap in the longitudinal direction between the device cap 20 with the connected cover sleeve 30 and the housing 3 allowing the device cap 20 with the cover sleeve 30 to travel a short distance between 0.5 to 5 mm. This may be necessary to absorb a force, for example, if the autoinjector 1 drops to the floor or to compensate for manufacturing tolerances.

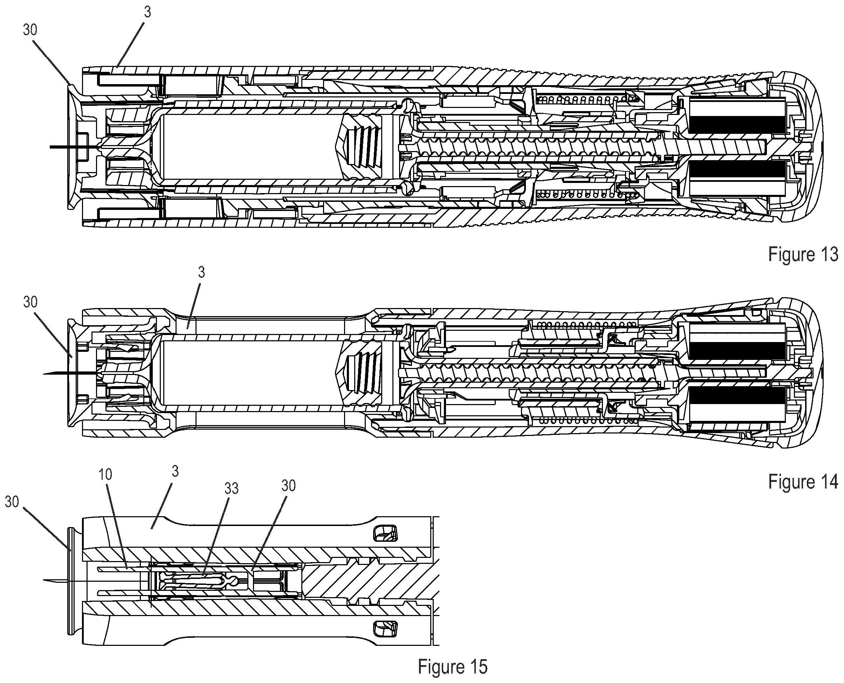

Figures 13 to 15 show again the same sectional views of the autoinjector 1 as figure 2 to 4 but in figures 13, 14 and 15 the autoinjector is in a dispensing state. That means the user has removed the device cap 20 and has placed the distal end of the cover sleeve 30 onto the injection site. Subsequently, the housing 3 has been pressed towards the injection site and thus the cover sleeve 30 has been moved proximally into the housing 3 into a retracted position.

That means the cover sleeve 30 was unlocked to be able to be moved from the covering position into the retracted position. As the uses has removed the device cap 20 the blocking ribs 26 with the blocking surfaces 27 were removed from the locking arms 33 meaning that the arms 33 have been released and have been free to deflect inwards from their initial position.

If the device cap blocking ribs 26are removed and if the cover sleeve 30 is pushed from the covering position towards the retracted position the knob end 34 of the locking arms 33 abut the proximal end of the rib 10 where the sloped surface 13 is arranged. As the knob end 34 of the flexible arm 33 has a sloped surface 34 too which is angled in the same direction as the rib sloped surface 13 the arms 33 are forced to deflect inwards towards a middle axis of the autoinjector in a plane tangent to the outer surface of the cover sleeve 30. The cover sleeve 30 is thus unlocked and free to move in longitudinal direction. If the cover sleeve 30 is further pushed in proximal direction the inwardly deflected arms 33 are guided along the longitudinal rib 10 by its knob ends 34. As best seen in figure 15 the inwardly deflected arms 33 are in-between the two longitudinal ribs 10 and the cover sleeve 30 is in its proximal end position, namely the retracted position. The elongate rib 10 guides the knobs 34 when the cover sleeve 30 returns to the covering or finally extended position, but does not prevent repeated retraction thereof (in the absence of blocking ribs 27).

The proximally moved cover sleeve 30 triggers the release of the drive spring 6 which in turn rotates the threaded rod 5. As the threaded rod 5 is in threaded engagement with the plunger rod 4 the latter is moved distally without rotation as it is non-rotatable guided by a housing part. The plunger rod 4 moves the piston 8 distally to dispense the liquid drug from the syringe 7 through the injection needle.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | |
|---|---|---|---|
| 1 | Autoinj ector | 30 | Needle cover sleeve |
| 2 | Proximal housing | 31 | Sleeve-shaped portion |
| 3 | Distal housing | 32 | Shell |
| 4 | Plunger rod | 33 | Flexible locking arms |
| 5 | Threaded rod | 34 | Knob end |
| 6 | Drive spring | 35 | Sloped surface |
| 7 | Syringe | 36 | opening |
| 8 | Piston | | |
| 9 | Needle | 40 | Rigid needle shield (RNS) |
| 10 | Rib | | |
| 11 | Support | 50 | Telescopic outer sleeve |
| 12 | Stop surface | 51 | Connecting surface |
| 13 | Sloped surface | 52 | cam |
| 14 | Cover sleeve spring | | |
| 15 | Opening | | |
| 16 | Housing gripping structure | | |
| 17 | Recess | | |
| 18 | Snapper | | |
| 19 | Nose | | |
| 20 | Device cap | | |
| 21 | Support structure | | |
| 22 | RNS remover sleeve | | |
| 23 | Hooks | | |
| 24 | Arms | | |
| 25 | Protrusion | | |
| 26 | Blocking rib | | |
| 27 | Blocking surface/cam surface | | |
| 28 | Cap gripping structure | | |
| 29 | Rim | | |

## Claims

1. A needle cover lock for an autoinjector (1) adapted to automatically dispense a liquid product through a needle (9) of a prefilled syringe (7), the needle cover lock comprising
• a housing (3) adapted to hold the syringe (7) and defining a longitudinal direction;
• a needle cover sleeve (30) movable relative to the housing (3) between an extended position and a retracted position;
• a device cap (20) attached to a distal end of the autoinjector (1) and covering a distal end of the needle
cover sleeve (30) in an unused state and removable by the user before using the autoinjector (1);
wherein the housing (3) or the needle cover sleeve (30) includes a flexible locking member (33) and the other of the housing (3) and needle cover sleeve (30) includes a stop surface (12) (13),
and wherein the device cap (20) includes a blocking surface (27) and wherein when the device cap (20) is attached, the blocking surface (27) together with the stop surface (12, 13) are adapted to prevent a deflection of the locking member (33) and thereby the needle cover sleeve (30) is prevented to exit the extended position wherein when the device cap (20) with its blocking surface (27) is removed from the autoinjector (1) the locking member (33) is allowed to deflect thereby allowing the needle cover sleeve (30) to exit the extended position and to move towards the retracted position **characterized in that** the locking member (33) is deflectable in a plane tangent to a circumferential outer surface of the needle cover sleeve (30) if the device cap (20) is removed.

2. Needle cover lock according to claim 1, wherein the stop surface (12) is arranged on a rib (10), which extends along the longitudinal direction and which is adapted to guide the flexible locking member (33) during a relative movement of the locking member (33) relative to the rib (10).

3. Needle cover lock according to claim 2, wherein the rib (10) includes a sloped surface (13) on a distal end of the rib adapted to facilitate deflection of the locking member (33) upon movement of the locking member (33) relative to the rib (10).

4. Needle cover lock according to any of claims 1 to 3, wherein the device cap (20) includes a blocking rib (26) extending in the longitudinal direction and wherein the blocking surface (27) is arranged on a longitudinal side of the blocking rib (26).

5. Needle cover lock according to claim 4, wherein the blocking rib (26) is arranged on an inside of the device cap (20).

6. Needle cover lock according to any of claims 1 to 5, wherein the locking member (33) has a sloped contact (35) surface to facilitate deflection of the locking member (33) upon movement of the locking member (33) relative to the stop surface (12) if the needle cover sleeve (30) is moved to exit the extended position.

7. Needle cover lock according to any of claims 1 to 6, wherein the locking member (33) is provided by the needle cover sleeve (30) and integrally formed in the needle cover sleeve (30).

8. Needle cover lock according to any of claims 1 to 7, wherein the locking member (33) is a flexible arm extending in the longitudinal direction.

9. Needle cover lock according to claim 8, wherein the flexible arm (33) includes a knob-shaped portion (34) at a free end of the arm.

10. Needle cover lock according to claim 8 or 9, including a first and second flexible arm (33) parallel arranged to each other and wherein the device cap (20) includes a first blocking surface (27) for the first arm (33) and second blocking surface (27) for the second arm (33) wherein the first and second blocking surfaces are oppositely arranged to each other and in-between the two parallel arms (33) if the device cap (20) is attached.

11. Needle cover lock according to any of claims 1 to 10, wherein in the extended position the attached device cap (20) and the needle cover sleeve (30) are movable together relative to the housing (3), preferably less than 5mm.

12. Needle cover lock according to any of claims 1 to 11, wherein the needle cover sleeve (30) is biased by a cover spring (14) towards the extended position.

13. Autoinjector (1) comprising the needle cover lock according to claims 1 to 12.

14. Autoinjector (1) according to claim 13, wherein the housing (3) is adapted to hold the syringe axially non-movable relative to the housing (3).

## Patentansprüche

1. Nadelabdeckungsverriegelung für einen Autoinjektor (1), der angepasst ist, um ein flüssiges Produkt durch eine Nadel (9) einer vorgefüllten Spritze (7) automatisch abzugeben, die Nadelabdeckungsverriegelung umfassend
• ein Gehäuse (3), das angepasst ist, um die Spritze (7) zu halten, und eine Längsrichtung definiert;
• eine Nadelabdeckhülse (30), die relativ zu dem Gehäuse (3) zwischen einer ausgefahrenen Position und einer zurückgezogenen Position bewegbar ist;
• eine Vorrichtungskappe (20), die an einem distalen Ende des Autoinjektors (1) angebracht ist und ein distales Ende der Nadelabdeckhülse (30) in einem unbenutzten Zustand abdeckt und vor einem Verwenden des Autoinjektors (1) durch den Benutzer entfernbar ist;
wobei das Gehäuse (3) oder die Nadelabdeckhülse (30) ein flexibles Verriegelungselement (33) einschließt und das andere von dem Gehäuse (3) und der Nadelabdeckhülse (30) eine Anschlagoberfläche (12) (13) einschließt,
und wobei die Vorrichtungskappe (20) eine Blockieroberfläche (27) einschließt und wobei, wenn die Vorrichtungskappe (20) angebracht ist, die Blockieroberfläche (27) zusammen mit der Anschlagoberfläche (12, 13) angepasst sind, um eine Auslenkung des Verriegelungselements (33) zu verhindern, und dadurch verhindert wird, dass die Nadelabdeckhülse (30) die ausgefahrene Position verlässt
wobei, wenn die Vorrichtungskappe (20) mit ihrer Blockieroberfläche (27) von dem Autoinjektor (1) entfernt wird, dem Verriegelungselement (33) ermöglicht wird, auszulenken, wodurch der Nadelabdeckungshülse (30) ermöglicht wird, die ausgefahrene Position zu verlassen und sich in Richtung der zurückgezogenen Position zu bewegen, **dadurch gekennzeichnet, dass** das Verriegelungselement (33) in einer Ebene auslenkbar ist, die tangential zu einer umlaufenden Außenoberfläche der Nadelabdeckungshülse (30) verläuft, falls die Vorrichtungskappe (20) entfernt ist.

2. Nadelabdeckungsverriegelung nach Anspruch 1, wobei die Anschlagoberfläche (12) an einer Rippe (10) angeordnet ist, die sich entlang der Längsrichtung erstreckt und die angepasst ist, um das flexible Verriegelungselements (33) während einer Relativbewegung des Verriegelungselements (33) relativ zu der Rippe (10) zu führen.

3. Nadelabdeckungsverriegelung nach Anspruch 2, wobei die Rippe (10) eine geneigte Oberfläche (13) an einem distalen Ende der Rippe einschließt, die angepasst ist, um die Auslenkung des Verriegelungselements (33) bei Bewegung des Verriegelungselements (33) relativ zu der Rippe (10) zu erleichtern.

4. Nadelabdeckungsverriegelung nach einem der Ansprüche 1 bis 3, wobei die Vorrichtungskappe (20) eine Blockierrippe (26) einschließt, die sich in der Längsrichtung erstreckt, und wobei die Blockieroberfläche (27) an einer Längsseite der Blockierrippe (26) angeordnet ist.

5. Nadelabdeckungsverriegelung nach Anspruch 4, wobei die Blockierrippe (26) an einer Innenseite der Vorrichtungskappe (20) angeordnet ist.

6. Nadelabdeckungsverriegelung nach einem der Ansprüche 1 bis 5, wobei das Verriegelungselement (33) eine geneigte Oberfläche für einen Kontakt (35) aufweist, um die Auslenkung des Verriegelungselements (33) bei Bewegung des Verriegelungselements (33) relativ zu der Anschlagoberfläche (12) zu erleichtern, falls die Nadelabdeckungshülse (30) bewegt wird, um die ausgefahrene Position zu verlassen.

7. Nadelabdeckungsverriegelung nach einem der Ansprüche 1 bis 6, wobei das Verriegelungselement (33) durch die Nadelabdeckungshülse (30) bereitgestellt und in der Nadelabdeckungshülse (30) einstückig ausgebildet ist.

8. Nadelabdeckungsverriegelung nach einem der Ansprüche 1 bis 7, wobei das Verriegelungselement (33) ein flexibler Arm ist, der sich in der Längsrichtung erstreckt.

9. Nadelabdeckungsverriegelung nach Anspruch 8, wobei der flexible Arm (33) einen knopfförmigen Abschnitt (34) an einem freien Ende des Arms einschließt.

10. Nadelabdeckungsverriegelung nach Anspruch 8 oder 9, umfassend einen ersten und einen zweiten flexiblen Arm (33), die parallel zueinander angeordnet sind, und wobei die Vorrichtungskappe (20) eine erste Blockieroberfläche (27) für den ersten Arm (33) und eine zweite Blockieroberfläche (27) für den zweiten Arm (33) umfasst, wobei die erste und zweite Blockieroberfläche einander gegenüberliegend und zwischen den zwei parallelen Armen (33) angeordnet sind, falls die Vorrichtungskappe (20) angebracht ist.

11. Nadelabdeckungsverriegelung nach einem der Ansprüche 1 bis 10, wobei in der ausgefahrenen Position die angebrachte Vorrichtungskappe (20) und die Nadelabdeckungshülse (30) zusammen relativ zu dem Gehäuse (3) bewegbar sind, vorzugsweise weniger als 5 mm.

12. Nadelabdeckungsverriegelung nach einem der Ansprüche 1 bis 11, wobei die Nadelabdeckungshülse (30) durch eine Abdeckungsfeder (14) in Richtung der ausgefahrenen Position vorgespannt ist.

13. Autoinjektor (1), umfassend die Nadelabdeckungsverriegelung nach den Ansprüchen 1 bis 12.

14. Autoinjektor (1) nach Anspruch 13, wobei das Gehäuse (3) angepasst ist, um die Spritze relativ zu dem Gehäuse (3) axial unbewegbar zu halten.

## Revendications

1. Verrou de protège-aiguille pour un auto-injecteur (1) conçu pour administrer automatiquement un produit liquide par l'intermédiaire d'une aiguille (9) d'une seringue préremplie (7), le verrou de protège-aiguille comprenant
• un logement (3) conçu pour loger la seringue (7) et définissant une direction longitudinale ;
• un manchon de protège-aiguille (30) mobile par rapport au logement (3) entre une position déployée et une position rétractée ;
• un capuchon de dispositif (20) fixé à une extrémité distale de l'auto-injecteur (1) et recouvrant une extrémité distale du manchon de protège-aiguille (30) dans un état non utilisé, et pouvant être retiré par l'utilisateur avant l'utilisation de l'auto-injecteur (1) ;
dans lequel le logement (3) ou le manchon de protège-aiguille (30) comporte un élément de verrouillage flexible (33) et l'autre parmi le logement (3) et le manchon de protège-aiguille (30) comporte une surface de butée (12) (13),
et dans lequel le capuchon de dispositif (20) comporte une surface de blocage (27) et dans lequel, lorsque le capuchon de dispositif (20) est fixé, la surface de blocage (27) conjointement avec la surface de butée (12, 13), sont conçues pour empêcher une déflexion de l'élément de verrouillage (33) et, de ce fait, le manchon de protège-aiguille (30) est empêché de quitter la position déployée
dans lequel, lorsque le capuchon de dispositif (20), avec sa surface de blocage (27), est retiré de l'auto-injecteur (1), l'élément de verrouillage (33) est autorisé à fléchir, permettant de ce fait au manchon de protège-aiguille (30) de quitter la position déployée et de se déplacer vers la position rétractée, **caractérisé en ce que** l'élément de verrouillage (33) peut fléchir dans un plan tangent à une surface extérieure circonférentielle du manchon de protège-aiguille (30) si le capuchon de dispositif (20) est retiré.

2. Verrou de protège-aiguille selon la revendication 1, dans lequel la surface de butée (12) est agencée sur une nervure (10) qui s'étend le long de la direction longitudinale et qui est conçue pour guider l'élément de verrouillage flexible (33) lors d'un mouvement relatif de l'élément de verrouillage (33) par rapport à la nervure (10).

3. Verrou de protège-aiguille selon la revendication 2, dans lequel la nervure (10) comporte une surface inclinée (13) sur une extrémité distale de la nervure, conçue pour faciliter la flexion de l'élément de verrouillage (33) lors d'un mouvement de l'élément de verrouillage (33) par rapport à la nervure (10).

4. Verrou de protège-aiguille selon l'une quelconque des revendications 1 à 3, dans lequel le capuchon de dispositif (20) comporte une nervure de blocage (26) s'étendant dans la direction longitudinale et dans lequel la surface de blocage (27) est agencée sur un côté longitudinal de la nervure de blocage (26).

5. Verrou de protège-aiguille selon la revendication 4, dans lequel la nervure de blocage (26) est agencée sur une face intérieure du capuchon de dispositif (20).

6. Verrou de protège-aiguille selon l'une quelconque des revendications 1 à 5, dans lequel l'élément de verrouillage (33) a une surface de contact inclinée (35) pour faciliter la flexion de l'élément de verrouillage (33) lors d'un mouvement de l'élément de verrouillage (33) par rapport à la surface de butée (12) si le manchon de protège-aiguille (30) est déplacé pour quitter la position déployée.

7. Verrou de protège-aiguille selon l'une quelconque des revendications 1 à 6, dans lequel l'élément de verrouillage (33) est fourni par le manchon de protège-aiguille (30) et est formé d'une seule pièce dans le manchon de protège-aiguille (30)

8. Verrou de protège-aiguille selon l'une quelconque des revendications 1 à 7, dans lequel l'élément de verrouillage (33) est un bras flexible s'étendant dans la direction longitudinale.

9. Verrou de protège-aiguille selon la revendication 8, dans lequel le bras flexible (33) comporte une partie en forme de bouton (34) à une extrémité libre du bras.

10. Verrou de protège-aiguille selon la revendication 8 ou 9, comportant un premier et un second bras flexible (33) agencés parallèlement l'un à l'autre, et dans lequel le capuchon de dispositif (20) comporte une première surface de blocage (27) pour le premier bras (33) et une seconde surface de blocage (27) pour le second bras (33), dans lequel les première et seconde surfaces de blocage sont agencées de manière opposée l'une à l'autre et situées entre les deux bras parallèles (33) si le capuchon de dispositif (20) est fixé.

11. Verrou de protège-aiguille selon l'une quelconque des revendications 1 à 10, dans lequel, dans la position déployée, le capuchon de dispositif (20) fixé et le manchon de protège-aiguille (30) sont mobiles ensemble par rapport au logement (3), de préférence sur une course inférieure à 5 mm.

12. Verrou de protège-aiguille selon l'une quelconque des revendications 1 à 11, dans lequel le manchon de protège-aiguille (30) est sollicité vers la position déployée par un ressort de protège-aiguille (14).

13. Auto-injecteur (1) comprenant le verrou de protège-aiguille selon les revendications 1 à 12.

14. Auto-injecteur (1) selon la revendication 13, dans lequel le logement (3) est conçu pour maintenir la seringue de manière axialement immobile par rapport au logement (3).
